(19) Europäisches Patentamt
European Patent Office
Office européen des brevets

(11) Publication number: **0 410 182 A2**

# (12) EUROPEAN PATENT APPLICATION

(21) Application number: **90112937.9**

(22) Date of filing: **06.07.90**

(51) Int. Cl.[5]: **C07K** 1/10, C07D 207/46, C07D 209/48, C07D 211/86, C07D 231/18, C07D 231/56, C07D 249/18, C07D 253/07

(30) Priority: **07.07.89 US 376715**

(43) Date of publication of application: **30.01.91 Bulletin 91/05**

(84) Designated Contracting States: **AT BE CH DE DK ES FR GB GR IT LI LU NL SE**

(71) Applicant: **Research Corporation Technologies, Inc**
**6840 East Broadway Boulevard**
**Tucson Arizona 85710-2815(US)**

(72) Inventor: **Carpino, Louis A.**
**11 Mount Pleasant**
**Amherst, Massachusetts 01003(US)**
Inventor: **Chao, Hann-Guang**
**113 Village Park**
**Amherst, Massachusetts 01002(US)**
Inventor: **Beyermann, Michael**
**Allee-der Kosmonauten 88**
**DD-1140 Berlin(DE)**

(74) Representative: **Hansen, Bernd, Dr. Dipl.-Chem. et al**
**Hoffmann, Eitle & Partner Patent- und Rechtsanwälte Arabellastrasse 4e 4**
**D-8000 München 81(DE)**

## (54) A new technique for rapid peptide coupling.

(57) The present invention is directed to a process for forming a peptide bond between a first AMINO ACID containing a free N-terminal amino group and an acylating N-terminal amino protected second AMINO ACID which comprises reacting under amide forming conditions said first AMINO ACID with said second AMINO ACID in the presence of a basic and catalytic effective amount of a mixture comprising a tertiary amine of the formula:

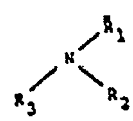

and a N-hydroxy nitrogen containing heterocycle or the quaternary ammonium salt of said mixture, wherein $R_1$, $R_2$, and $R_3$ are independently lower alkyl, or $R_1$ and $R_2$ together with the nitrogen to which they are attached form a heterocyclic ring.

EP 0 410 182 A2

# A NEW TECHNIQUE FOR RAPID PEPTIDE COUPLING

The present invention relates to a new process for effecting the acylation step in peptide synthesis. More specifically, the invention relates to a novel mixture of a N-hydroxy-nitrogen containing heterocycle and a tertiary amine or quaternary ammonium salt for use as a base catalyst in the acylation step in peptide synthesis.

As more and more polypeptides become of medicinal importance, there is an increasing incentive to improve the methods by which they may be synthesized. In recent years, peptides which have been found to be of possible pharmacological importance include those active against various diseases, such as cancers, diabetes, and plant toxins, etc. Others have shown specific activity as growth promoters or suppressants, antibiotics, insecticides, contraceptives, anti-hypertensives, sleep-inducers, anti-depressants, analgesics, etc. The list is long and varied.

Currently synthesis of peptides in solution by classic or various repetitive methods or on a solid support (Merrifield) are popular techniques. Solution methods have the advantage of being easily monitored and allowing purification of intermediates, if necessary, at any stage. A major drawback is the relative slow pace of the synthesis with each step being carried out manually.

The major advantage of the Merrifield Method is its easy automation so that unattended, computer-controlled machine syntheses is possible. Unfortunately, the method suffers from an inherent deficiency due to the insoluble nature of the support on which the synthesis proceeds. Unless each acylation step occurs with 100% efficiency, mixtures will inevitably be built up on the polymer. The longer the chain, the greater will be the contamination by undesired side reactions. Products produced in such reactions remain to contaminate the desired product when at the end of the cycle it is removed from the polymeric matrix. The properties of these peptides will not differ sufficiently for peptides of greater than about 20-30 residues to make efficient separation feasible.

For very long segments (50 or more amino acids), therefore, current methods are not satisfactory. Often mixtures are obtained of such forbidding complexity that it may be difficult or impossible to isolate the desired peptide.

The problems enumerated herinabove could be eliminated if the proper derivatives of the underlying amino acids and the proper reaction conditions could be found. Protecting groups, such as N $\alpha$-(9-fluorenylmethyl)- oxycarbonyl (Fmoc), have been used to minimize side reactions. But, additionally, other aspects of the coupling reaction must also be taken into consideration such as the acylating moiety of the N-protected amino acids as well as the reaction conditions for the coupling reaction to proceed. Emphasis has been placed in finding the proper reaction conditions for forming a relatively pure peptide, with little, if any racemization product being formed from inexpensive and readily available reagents in a fast and efficient manner.

In Fmoc based solid phase peptide synthesis, Fmoc amino acid pentafluorophenyl esters (Fmoc-AA-Opfp), the analogue N-hydroxy benzotriazene esters and symmetrical anhydrides are being used. Yet, these esters are expensive, and the use of the symmetrical anhydrides wastes one half of the active reagent.

A candidate for the coupling reagent is Fmoc-amino acid chlorides which were described and isolated by Carpino et al., J . Org. Chem . 51 , 3732 (1986). This reagent is easy to prepare and its reactivity is high. Carpino, et al., utilized said reagent in the synthesis of a peptide in solution and noticed no racemization to occur. However, one of the drawbacks of this reagent is that HCl, which is generated during the reaction, needs to be neutralized. An aqueous solution of an inorganic base such as sodium carbonate would neutralize the HCl, but the addition of an aqueous solution is not miscible with the organic phase. Therefore, an inorganic base cannot be used in synthesizing peptides in a homogeneous organic solution, as in solid phase peptide synthesis.

However, the use of the mixture of the present invention described hereinbelow in peptide synthesis overcomes many of these problems, because the products formed are purer and higher quality, than those formed by methods here before. No racemization is detected, using the process of the present invention. The reaction conditions are very mild and the reagents used are commercially available and/or easy to prepare. And, the mixture used in the present process is easily adaptable for use in solid phase peptide synthesis.

Moreover, the present invention serves to speed the course of solid phase peptide synthesis. In addition, the time savings due to decreased cycle terms can be substantial for long sequences. Thus a 60-MER which normally might require three days for assembly could now be available in a short period of time, such as a day or day and a half. Furthermore, as mentioned above, additional time savings would follow due to increased ease of purification of the initial crude product. As indicated herinabove, solid phase

syntheses are used to obtain segments which are first purified and then assembled in solution or on solid phase to give longer peptides. Such techniques often lead to peptides which are easier to purify than those obtained by the continuous, stepwide procedure. The process of the present process allows the practitioner in the field to synthesize peptide segments containing 10-15 amino acids which do not require further purification for subsequent segment condensation.

The present invention relates to a process for forming a peptide bond between a first AMINO ACID containing a free N-terminal amino group and an acylating N-terminal amino protected second AMINO ACID which comprises reacting under amide forming conditions said first AMINO ACID with said second AMINO ACID in the presence of a basic and catalytic effective amount of a mixture comprising a tertiary amine of the formula:

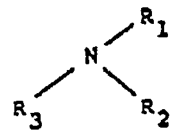

and a N-hydroxy nitrogen containing heterocycle or the quaternary ammonium salt of said mixture, wherein $R_1$, $R_2$, and $R_3$ are independently lower alkyl, or $R_1$ and $R_2$ together with the nitrogen to which they are attached form a heterocyclic ring.

The present invention further relates to a process for preparing a peptide which comprises:

(a) reacting a first AMINO ACID containing a free N-terminal amino group and an acylating N -amino protected amino acid under amide forming conditions in the presence of basic and catalytically effective amount of a tertiary amine of the formula:

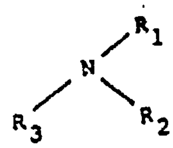

and a N-hydroxy nitrogen containing heterocycle or the quaternary ammonium salt of said mixture, wherein

$R_1$, $R_2$, $R_3$ are independently lower alkyl, or $R_1$ and $R_2$ together with the nitrogen to which they are attached form a heterocyclic ring;

(b) removing the Nα-protecting groups from the amino acid residue;

(c) repeating steps (a) and (b) until the desired peptide has been obtained.

The present invention further relates to a quaternary ammonium salt comprising as a base, an oxide of a N-hydroxy containing heterocycle and as the acid, a quaternary ammonium cation of the formula:

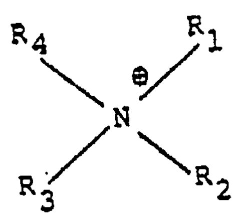

wherein $R_1$ and $R_2$ and $R_3$ are independently lower alkyl or $R_1$ and $R_2$ taken together with the nitrogen to which they are attached form a heterocyclic ring; and

$R_4$ is hydrogen, or alkyl containing from 1 to 20 carbon atoms.

The present invention is directed to the synergestic effect of the tertiary amine and the N-hydroxy-nitrogen containing heterocycle in effecting the formation of a peptide bond betweeen a first AMINO ACID with a free N-terminal amino group and an acylating N-terminal amino protected second AMINO ACID. The heterocycle by itself or the tertiary amine by itself is virtually ineffective; but the combination is superbly effective.

As employed herein, the expression nitrogen containing heterocycle is meant to include those

heterocyclic rings which have at least one ring nitrogen atom. However, other hetero atoms, such as sulfur or oxygen may additionally be present on the ring. It is preferred, however, that the heterocyclic ring contain at least 1 nitrogen ring atom and the most preferred heterocyclic ring contains at least 2-nitrogen ring atoms. The heterocyclic moiety may be fully saturated, partially unsaturated, or heteroaromatic. The heterocyclic ring contains from 5 to 14 ring atoms and up to a total of 13 ring carbon atoms and a total of 18 carbon atoms. The heterocyclic ring may be monocyclic, bicyclic or tricyclic. Also included in this expression are the so-called "benzo" heterocycles. Examples include benzotriazole, pyrrole, indazole, imidazole, pyrazole, pyrazines, piperidine, pyrimidine, pyridazine, indole, purine, isoquinoline, quinoline, quinoxaline, quinazoline, carbazole, furazan, pthalimide, and the like.

As used herein, the term N-hydroxy nitrogen containing heterocycle refers to the nitrogen containing heterocycle defined hereinabove substituted by an hydroxy on one of the ring nitrogen atoms.

The preferred N-hydroxy nitrogen containing heterocycles have the formula:

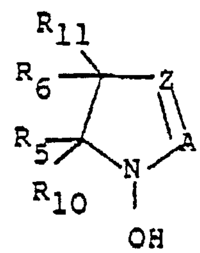
,
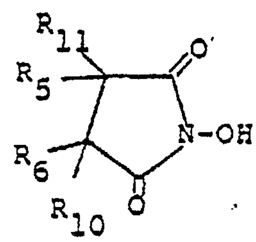
,

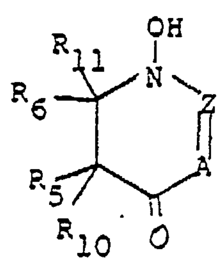
,
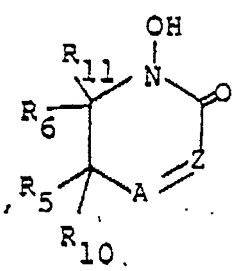
, or

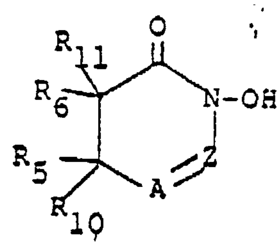

wherein

$R_5$ and $R_6$ are independently hydrogen or lower alkyl or $R_6$ and $R_5$ taken together with the carbon atoms to which they are attached form an aromatic ring which is unsubstituted or monosubstituted with lower alkyl or an electron withdrawing group;

A is N or $CR_7$;

Z is N or $CR_8$ and A is N when Z is N; and

$R_7$ and $R_8$ are independently lower alkyl, hydrogen or an electron withdrawing group;

$R_{10}$ and $R_{11}$ are hydrogen or lower alkyl or an electron withdrawing group or $R_{10}$ and $R_{11}$ taken together form a bond or $R_{10}$ and $R_{11}$ are when $R_5$ are taken together form an aromatic ring. In other words, when $R_5$ and $R_6$ taken together form an aromatic ring, such as benzo, $R_{11}$ and $R_{10}$ are not present. On the other hand, when $R_{10}$ and $R_{11}$ form a bond, a double bond is present in said heterocyclic at the corresponding positions. For example, heterocycles such as

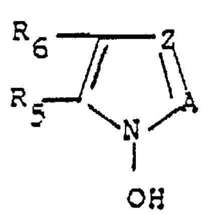, 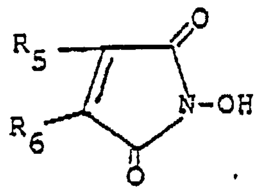,

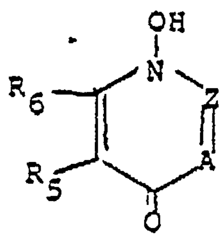, 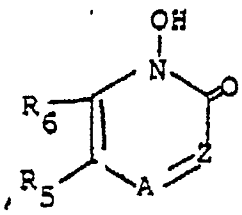, or

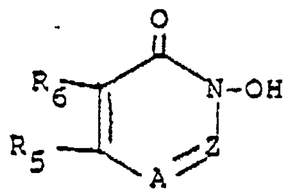

wherein

Z, A are as defined above and $R_6$ and $R_5$ are independently hydrogen lower alkyl or an electron withdrawing group are contemplated to be within the scope of N-hydroxy heterocycles used in the present invention.

The most preferred N-hydroxy nitrogen containing heterocycles have the formula:

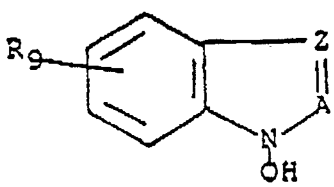, 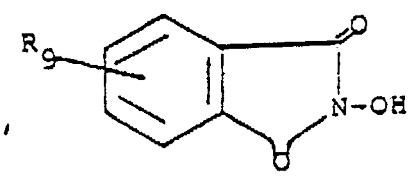,

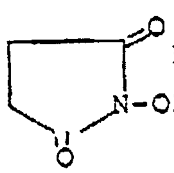, 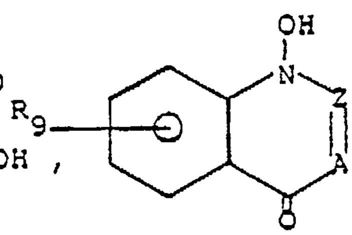, 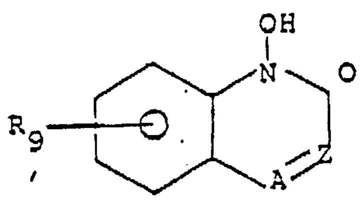, or

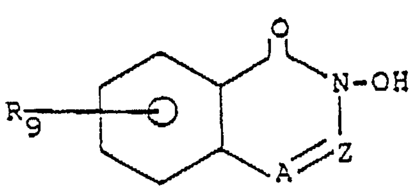

wherein Z A, $R_7$, and $R_8$ are as defined hereinabove and $R_9$ is hydrogen, lower alkyl or an electron withdrawing group.

Especially preferred N-hydroxy nitrogen containing heterocycles have the formula

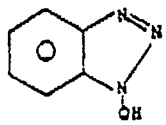 , 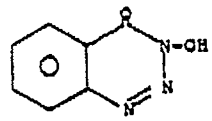 , 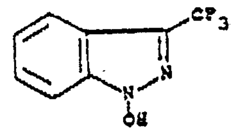 ,

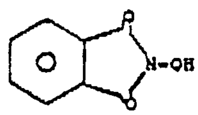 or 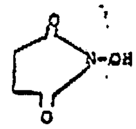

The most especially preferred N-hydroxy heterocycles is hydroxybenzotriazole (HOBt).

As defined herein, the term lower alkyl, when used singly or in combination, refer to alkyl groups containing one to six carbon atoms. They may be straight chain or branched and include such groups as methyl, ethyl, propyl, isopropyl, butyl, sec-butyl, isobutyl, t-butyl, pentyl, isopentyl, neopentyl, hexyl and the like. The preferred alkyl groups contain one to four carbon atoms.

The term aromatic ring when used alone or in combination refers to an aryl ring which contains 6 to 10 ring carbon atoms. These groups include phenyl, α-naphthyl, β-naphthyl, and the like. The preferred aromatic ring is phenyl.

Therefore, it is preferred that when $R_5$ and $R_6$ are taken together with the carbon atoms to which they are attached, they form a "benzo" ring.

The term electron withdrawing group as defined herein shall be interpreted as a group that will draw electrons to itself more than a hydrogen atom would if it occupied the same position in the molecule. See , J. March, Advanced Organic Chemistry , 3rd Ed., John Wiley and Sons, Page 17 (1985). Moreover, as used herein, electron withdrawing groups shall not contain a labile or easily ionizable hydrogen, as these types of groups would interfere with the coupling reaction.

The type of groups referred to as electron withdrawing groups as defined herein are well known to one skilled in the art. They include such groups as nitro, monohalolkyl, dihaloalkyl, trihaloalkyl, e.g., (trifluoromethyl), halo, formyl, lower alkanoyl, lower alkylsulfonyl, lower alkylsulfinyl, oxo, and the like. The preferred electron withdrawing groups are nitro and trifluoromethyl.

Halo as used herein included chloro, bromo, iodo and especially fluoro.

The tertiary amine is the other reagent which is used in the mixture as defined in the present invention. The tertiary amine is defined by the structure

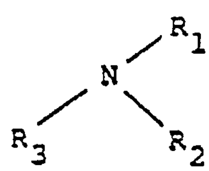

wherein $R_1$, $R_2$, and $R_3$ are as defined herein. As defined herein, the tertiary amine can be a bicyclic diamino compound, such as DABCO. The preferred tertiary amines include diisopropyl ethyl amine, triethylamine, and N-methylmorpholine, DMAP, and DABCO.

The mixture, as contemplated herein, includes the N-hydroxy nitrogen containing heterocycle and the tertiary amine as defined hereinabove. Alternatively, the quaternary ammonium salts of this mixture can be used in the process of the present invention. The quaternary ammonium salts refer to salts comprising the N-oxide of the nitrogen containing heterocycle and the quaternary amine having the formula

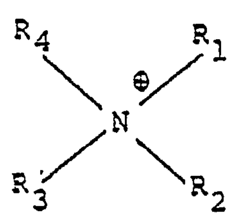

wherein $R_1$, $R_2$, and $R_3$ are as defined hereinabove and $R_4$ is hydrogen or $C_1$-$C_{20}$ alkyl, such as decyl, dodecyl, cetyl and the like.

Preferred quaternary ammonium salts have the formula:

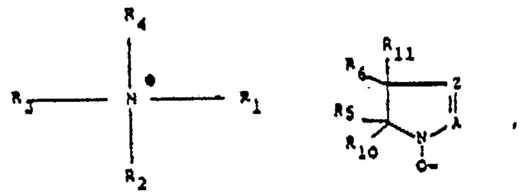

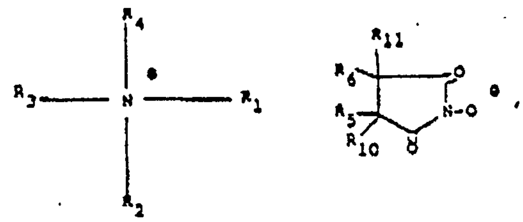

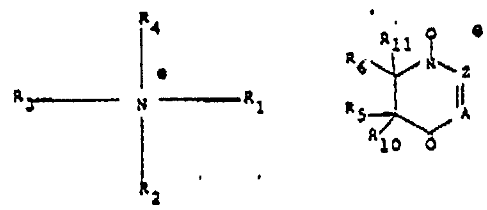

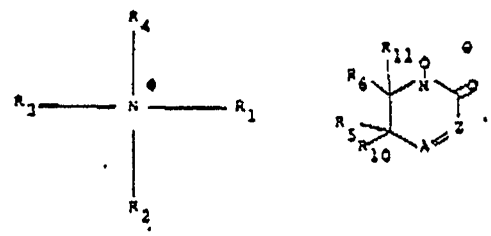

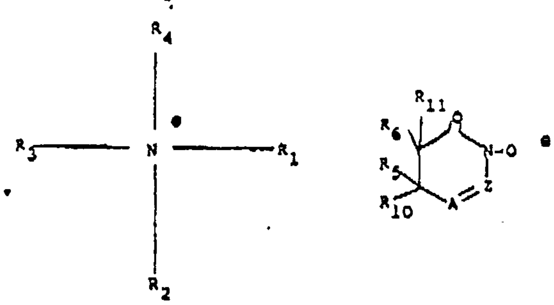

wherein $R_1$, $R_2$, $R_3$, $R_4$, $R_5$, $R_6$, A, Z, $R_7$, and $R_8$ are defined hereinabove.

The especially preferred salts of the mixture are those ammonium salts wherein $R_5$ and $R_6$ taken together with the carbon atoms to which they are attached form a phenyl ring as well as the N-oxide of the succinimide. These quaternary salts have the formula

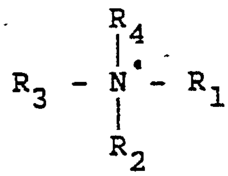 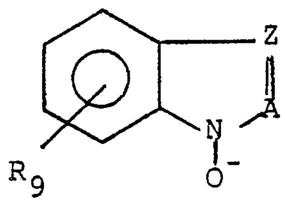

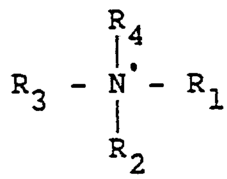 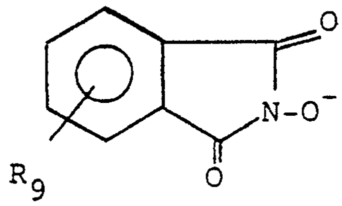

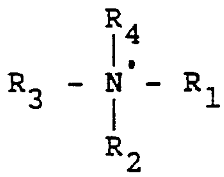 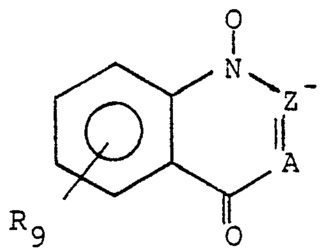

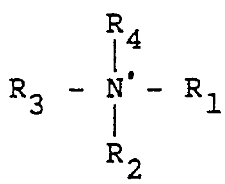 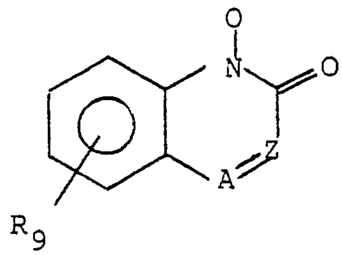

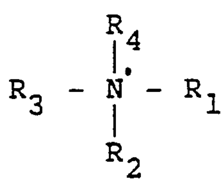 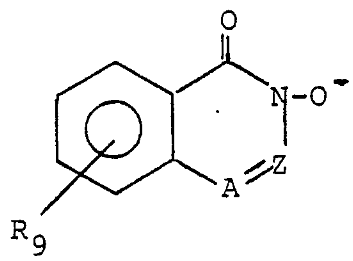

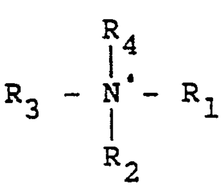 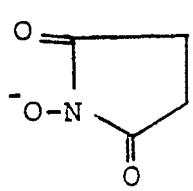

wherein $R_1$, $R_2$, $R_3$, $R_4$, $R_7$, $R_8$, $R_9$, $R_{10}$, $R_{11}$, A, and Z are as defined hereinabove. The most preferred salt of the mixture is $Et_4N^+$ $^-OBt$, or a salt of DMAP and HOBt; DMAP.HOOBt; DABCO.HOBt, DIEA.HOBt or triethyeamine.HOBt.

Both the tertiary amine and the N-hydroxy-nitrogen containing heterocycle used herein are known reagents which are commercially available or can be synthesized according to procedures known in the prior art.

The mixtures described herein can be used in the formation of any amide linkages from the coupling of an amine and a carboxylic acid or an acylating derivative thereof. However, their significance is best realized in the synthesis of bioorganic molecules, e.g., peptides, and polypeptides, nucleotides, and polynucleotides.

An application of the process of the present invention is using the mixture described herein in peptide synthesis.

The mixture described hereinabove can therefore be used to promote the coupling of a first AMINO ACID containing a free N-terminal amino group with an acylating N-terminal amino protected second AMINO ACID.

The process of the present invention is general; it can be used in effecting the coupling of two amino acids, but also can be used to effect the coupling of a dipeptide and an amino acid, a tripeptide and an amino acid, a tetrapeptide and an amino acid, tetrapeptide, pentapeptide, higher peptides, polypeptides and proteins. Therefore, to connote the generalized applicability of the process of the present invention, the term AMINO ACID, as used herein, is used as a general term, referring to an amino acid, or a dipeptide, tripeptide, tetrapeptide, or higher peptide or polypeptide or protein. The preferred second AMINO ACID is an -amino acid defined hereinabove.

The term amino acid refers to α-or •β amino acids and include the twenty naturally occurring amino acids. The term includes such amino acids as alanine, glycine, valine, leucine, isoleucine, proline, phenylalanine, tryptophan, methionine, serine, threonine, cysteine, tyrosine, asparagine, glutamine, aspartic acid, glutamic acid, lysine, arginine and histidine. It also includes cystine, β-alanine, as well as the synthetic amino acids, such as 3-(2-naphthyl) alanine. The preferred amino acids are the α-amino acids.

The synthesis of peptides normally followed synthetic sequences requiring five steps for the formation of each peptide bond. These are

1. Protection of the amino group on an amino acid;
2. Protection of the carboxyl group on another;
3. Activation of the carboxyl group on the first amino acid,
4. Formation of a peptide bond, and
5. Removal of the group protecting the amino group of the first amino acid.

A variety of carboxy protecting groups known in the art may be employed. Examples of many of these possible groups may be found in "Protective Groups in Organic Synthesis", by T.W. Green, John Wiley and Sons, 1981, which is incorporated herein by reference.

A number of blocking reagents for amino groups are known in the art and have been utilized in the synthesis of peptides. These blocking groups are discussed in U.S. Patent Nos. 3,835,175, 4,508,657, 3,839,396, 4,108,846 and the contents thereof are incorporated herein by reference as if fully set forth herein. Moreover, other amino protecting groups are discussed in copending application, U.S. Patent Application Serial No. 364,662 and incorporated herein by reference. Other amino protecting groups are described in an article entitled "Solid Phase Peptide Synthesis" by G. Barany and R.B. Merrifield in THE PEPTIDES , Vol 2, edited by E. Gross and J. Meienhofer, Academic Press, New York, NY, p. 100-118 (1980), the contents of which are incorporated herein by reference. Some protecting groups, such as benzyloxy carbonyl or t-butyloxycarbonyl are removed by acidolysis, others such as Fmoc, are removed by base, still others, such as 2-(t-butylsulfonyl)-2-propenyloxycarbonyl (BSPOC) or benzothiophene-sulfone-2-methyloxycarbonyl, are removed by nucleophiles while others are removed by catalytic hydrogenation, such as benzyloxy-carbonyl. The protected amino acids having protecting groups on the amino moiety are contemplated to be within the scope of the present invention.

To facilitate the coupling, the N-terminal amino protected AMINO ACID is an acylating derivative thereof. By acylating, it is meant that the AMINO ACID has a free carboxylic acid group; or is an acid halide; or ester, such as lower alkyl ester, phenoxy ester which is unsubstituted or substituted with 1-5 electron withdrawing groups as defined herein; or an anhydride and the like. The preferred acylating derivative is the amino acid chloride or the pentafluorophenyl ester.

The preferred acylating N-terminal amino protected second AMINO ACID is an amino acid of the formula

L-AA-X

wherein L is an amino protecting group, described hereinabove,

AA is an amino acid moiety defined hereinabove less the amino hydrogen and less the acyl hydroxy; and

X is hydroxy, halo or

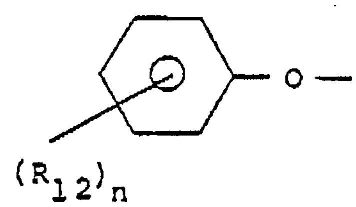

wherein each $R_{12}$ is independently halo, lower alkyl, nitro, cyano or other electron withdrawing group as defined herein and n is integer from 0-5. When n is 0, then the phenoxy ester is unsubstituted. The preferred second AMINO ACID is Fmoc AA chloride or Fmoc-AA-pentafluorophenyl ester where AA is one of the twenty naturally occuring amino acids.

The acylating N-terminal amino protected second amino acid can easily be prepared by art recognized techniques. Although the order is not important, if the amino acid is the starting material then the amino protecting group is usually first added to the AMINO ACID by procedures known in the art. Then the acylating group is added by procedures known in the art. For example, to form the acid halide, the amino protected amino acid is reacted with thionyl chloride or $PCl_3$ or $PCl_5$ by known techniques in the art to form the acid chloride; $PBr_3$ or $PBr_5$ to form the acid bromide; cyanuric fluoride to form the acid fluoride.

The AMINO ACID ester in turn can be formed by reacting the acid halide formed hereinabove with an alcohol. Alternatively, the ester can be prepared under Fischer esterification conditions by reacting the protected amino acid with the alcohol in the presence of an acid, such as paratoluenesulfonic acid. In the latter method, the protecting group that is used should not be acid sensitive.

The coupling of the AMINO ACID with a free amino group and the acylating N-α-protecting second AMINO ACID takes place in the presence of a basic and catalytic effective amount of the mixture of the N-hydroxy nitrogen containing heterocycle and a tertiary amine or the quaternary ammonium salt of the mixture. The heterocycle and the tertiary amine should be present in effective amounts, ranging from a molar ratio of heterocycle to amine ranging from 1:3 to 3:1. It is preferred that the heterocycle and amine should be present in approximately equimolar amounts.

With respect ot the AMINO ACIDS, the mixture should be present in basic and catalytic effective amounts. Without wishing to be bound, it is believed that the mixture acts both as a catalyst in the present invention as well as a base. It is believed that the reaction proceeds through the formation of the quaternary ammonium salts defined herein. Using HOBt as representative of the N-OH heterocycle, the mechanism is thought to proceed as follows:

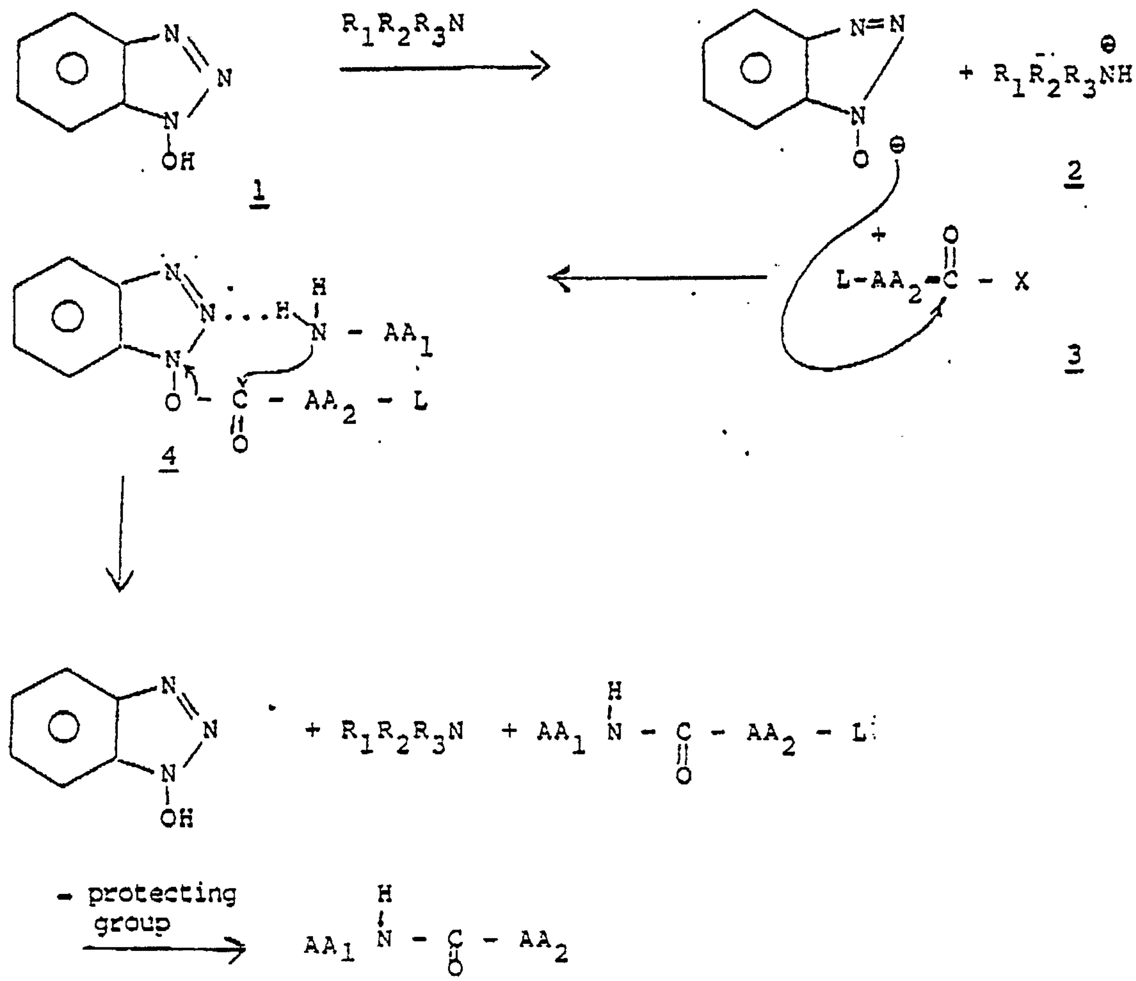


1 2 3 4

L is an amino protectant group
wherein $R_1$, $R_2$, $R_3$, X and L are as defined hereinabove;
$AA_1$ is the 1st amino acid less the amino group;
$AA_2$ as defined herein is the acylating N-protected α-amino second amino acid less the acyl group.

The N-hydroxy heterocycle forms a salt with the tertiary amine (2). Nucleophilic substitution on the acyl group of the second AMINO ACID (3) leads to product 4. The first amino acid hydrogen bonds to the nitrogen atoms on the heterocycle ring, which serves as an anchor and facilitates the coupling of the amino acids, thereby regenerating the heterocycle (1) and the tertiary amine and forming the dipeptide.

As shown by the scheme hereinabove, these quaternary ammonium salts are prepared by an acid-base reaction between the N-hydroxy heterocycle and a tertiary amine. This reaction may take place in an inert solvent, such as methylene chloride, methanol, ether, dimethylformamide and the like. Alternatively, the reaction can take place in situ .

It is preferred that the first and second AMINO ACIDS as well as the tertiary amine and the N-hydroxy heterocycle be present in approximately equimolar amounts, although up to about 3 molar equivalence of any of the reagents can also be employed.

The preferred second amino acid in this process are Fmoc AA chloride or Fmoc AA pentafluorophenyl ester. The preferred mixture is an equimolar mixture of N-hydroxybenzotriazole and diisopropyl ethyl amine. In place of the mixture, the preferred salts are $Et_4N^+$ $^-$OBt, DMAP.HOBT, salt, DMAP HOOBt salt, DABCO.HOOBt salt, DIEA.HOBt, or triethylamine.HOBt. Another preferred N-hydroxy heterocycle is the N-hydroxy indazole of the formula:

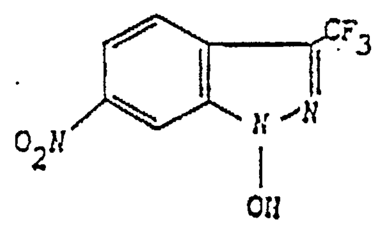

which also is effective.

Moreover, the corresponding quaternary ammonium salt of the N-hydroxy indazole hereinabove may have the cation moiety being $(DMAP)^+$, $(DIEA)^+$, $(DABCO)^+$; $NEt_4^+$ or $NEt_3H^+$.

The coupling reaction described hereinabove can take place in the additional presence of a dehydrating agent, such as DCC. The coupling reaction usually takes place in an inert polar solvent, such as dimethylformamide (DMF) or ethers. In fact, DMF is the preferred solvent in solid phase synthesis because of its favorable solvation properties. However, unlike the other systems, in the process of the present invention, no racemization is detected. The reaction takes place under mild temperature conditions usually ranging from about 15° C to about 30° C.

Therefore, the present invention is useful for preparing a peptide which comprises

(a) reacting a first AMINO ACID containing a free N-terminal amino group and an acylating N -amino protected second AMINO ACID under amide forming conditions in the presence of basic and catalytically effective amount of a tertiary amine

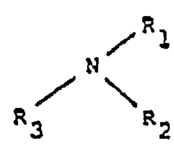

and a N-hydroxy nitrogen containing heterocycle or the quaternary ammonium salt of said mixture, wherein

$R_1$, $R_2$, $R_3$ are independently lower alkyl, or $R_1$ and $R_2$ together with the nitrogen to which they are attached form a heterocyclic ring;

(b) removing the N -amino protecting groups from the second amino acid residue;

(c) repeating steps (a) and (b) until the desired peptide has been obtained.

The present invention can be readily utilized in solid phase peptide synthesis. Solid phase peptide synthesis is based on the stepwise assembly of a peptide chain while it is attached at one end to a solid support or solid phase peptide resin. Two methods are generally well known in the art.

One, the Merrifield method, employs the solid support for attachment of the amino acid or peptide residues. This method employs N-protected amino acids as building blocks which are added to an amino acid or peptide residue attached to the solid support at the carbonyl (acid) end of the molecule. After the peptide bond has been formed, the protecting group is removed and the cycle repeated. When a peptide having the desired sequence has been synthesized, it is then removed from the support.

The second method, inverse Merrifield, employs reagents attached to solid supports in a series of columns. The amino acid or peptide residue is passed through these columns in series to form the desired amino acid sequence.

These methods are well known in the art and are discussed in U.S. Patent Nos. 4,108,846, 3,839,396, 3,835,175, 4,508,657, 4,623,484, 4,575,541, 4,581,167, 4,394,519 as well as in Advances In Enzymology, 32, 221 (1969) and in PEPTIDES, edited by Erhard Gross and Johannes Meienhofer, Vol 2, Academic Press, p. 3-255 (1980) and are incorporated herein by reference as if fully set forth herein.

An embodiment of this invention is the use of Fmoc amino acid chlorides (Fmoc-AA-Cl) as coupling agents. These compounds are potentially the cheapest coupling agents available since they can be synthesized readily on a large scale by reaction of the acid with thionyl chloride. The acid chlorides are shelf-stable, crystalline solids which can be easily purified and stored indefinitely in capsules or cartridges for eventual direct use on automated peptide synthesizers. By themselves, however, these acid chlorides are not superior to pfp esters since coupling to resin bound amino acid esters occurs surprisingly slowly under classical acylation conditions, e.g., in the presence of an organic base (pyridine, triethylamine, N-methylmorpholine, di-isopropyl ethyl amine, etc.) as both catalyst and hydrogen chloride acceptor. On the other hand, if an equimolar mixture of N-hydroxybenzotriazole (HOBt) and di-isopropyl ethyl amine (DIEA) is

used as HCl acceptor and catalyst, the coupling reaction takes place with remarkable rapidity. As an example, upon treatment of 200 mg of a Sheppard leucine resin (0.2 mmol/g) under normal batch conditions with a solution of Fmoc-Phe-Cl (4 eq) in the presence of a 1:1 mixture of HOBt and $iPR_2NEt$ (4 eq each) in DMF (final concentration 0.1 M) complete acylation is observed (Kaiser test) after 3 min.

## EXAMPLES

The invention will now be illustrated by examples. The examples are not intended to be limiting of the scope of the present invention. In conjunction with the general and detailed description above, the examples provide further understanding of the present invention.

## EXAMPLE 1

General Protocol for Test Coupling of FMOC Amino Acid Chlorides with a Leucine-Loaded Resin.

The FMOC-Leu-resin was a sample of Sheppard KA resin (0.1 meq/g) provided by Milligen Division, Millipore Corporation. The FMOC amino acid chlorides were all synthesized by described techniques [Carpino, et al., J. Org. Chem. 51, 3732 (1986)]. Test reactions were carried out manually in small syringes with mixing being accomplished by brief stirring by hand. The syringe was attached to a Millipore vacuum manifold to effect rapid removal of reagents and solvents. The FMOC-leucine resin was first washed with DMF, deblocked with piperidine and then acylated according to a standard 9-step protocol which is reproduced below. In each case for 200 mg of FMOC-leu-resin there was used 4 eqs of FMOC-AA-Cl, 4 eqs of either a t-amine or 4 eq s of a 1:1 mixture of HOBt/amine (0.16 M, 0.5 mL) in DMF. The acid chloride was dissoved in 0.3 mL of DMF to give a final concentration of or 0.1. Results are recorded in Table 1. The FMOC leucine resin contained 1.1-1.4% of the D-isomer based on GC analysis. When a valine resin was similarly acylated via FMOC-Val-Cl/HOBt/DIEA for 4 min. the Kaiser test indicated nearly complete acylation (trace of blue color).

### 9-Step Protocol

1) DMF wash 3 mL (1 min) x 2
2) 20% piperidine wash 2 mL (5 min) x 2
3) DMF wash 3 mL (1 min) x 3
4) DMF HOBt/DIEA wash 2 mL (1 min) x 1
5) Acylation (3 min)
6) DMF wash 3 mL (1 min) x 5
7) $CH_2Cl_2$ wash 3 mL (1 min) x 2
8) MeOH wash wash 3 mL (1 min) x 1
9) Ether wash 3 mL (1 min) x 3.

TABLE I

*Ease of Acylation and Question of Racemization*

*During Solid Phase Coupling Reactions*

| Fmoc-AA-Cl | Base | Acylation Time | Kaiser Test Results | Rac. (%)[a] |
|---|---|---|---|---|
| Phe | Pyridine | 5 min | + | |
| Phe | Di-t-Bu-Pyridine | 5 min | + | |
| Phe | Di-t-Bu-Pyridine | 10 min | + | |
| Phe | HOBt | 5 min | + | |
| Phe | DIEA | 5 min | + | |
| Phe | DIEA/SuOH | 5 min | + | |
| Phe | DIEA/PhtOH | 5 min | + | |
| Phe | $NEt_3$/HOBt | 5 min | - | 1.17[b] |
| Phe | $NEt_3$/HOBt | 3 min | - | 1.18[b] |
| Phe | DIEA/HOBt | 3 min | - | 1.21[b] |
| Phe | Pyr/HOBt | 3 min | + | |
| Phe | NMP/HOBt | 3 min | - | 1.40[b] |
| Phe | DMAP/HOBt | 3 min | + | |
| Phe | NMM/HOBt | 3 min | - | |
| Phe | DBN/HOBt | 3 min | sb[c] | |
| Phe | Pemp/HOBt | 3 min | sb[c] | |
| Phe | DIEA/HOBt[d] | 3 min | + | |
| Phe | DIEA/HOBt[d] | 5 min | + | |
| Val | DIEA/HOBt | 3 min | - | |

Table I. (Continued)

a) Not tested except where indicated

b) Racemization was determined by HPLC analysis. The figures given are averages for two independent runs. These results agree with the amount of DL-already present on the resin.

c) sb: slightly blue. Incomplete coupling reactions might be due to insufficient mixing.

d) Reaction run in methylene chloride.

Racemization Study.--200 mg of FMOC-Phe-Leu-resin obtained as described above was suspended in 5 mL of dry MeOH containing a few drops of conc. $H_2SO_4$. After refluxing for 4 h, the solution was allowed to cool to room temperature, filtered and solvent removed in vacuo. The residue was dissolved in 10 mL of EtOAc and washed with 10% $NaHCO_3$ (10mL x 3) and then saturated NaCl solution. Drying over $MgSO_4$ and removal of solvent gave the FMOC-Phe-Leu-OMe as a white solid (85-90%) along with a small amount of unidentified impurity (TLC). 2 Mg of the solid was dissolved in 5 ML of EtOAc and 5 mL of this solution was injected onto a Waters Radialpak 10u silica gel column (0.8 x 10cm) with elution by 99.2/0.8% hexane/i-PrOH, f=2, tR(LL) = 17.53±1 min and tR (LD or DL) 22.0± 1 min. Results are recorded in Table I.

Preparation of FMOC-Tyr-(t-Bu)-Cl--To a solution of 1g of FMOC-Tyr(t-Bu)-OH in 15 mL of THF at 0° C was added 1.5 eq of $SOCl_2$ and 0.2 eq of TMU (tetramethyl urea). The resulting solution was stirred at 0° C for 40 min., solvent removed in vacuo with the aid of vacuum pump to give a solid. Recrystallization from $CH_2Cl_2$-hexane gave 670 mg (66,7%) of the acid chloride as a white solid, mp 78-82° C; HPLC f=2, solvent MeOH/$H_2O$ 0.1% TFA 75:25, FMOC-Tyr-Cl (methyl ester) tR=5.33 (0.53%), FMOC-Tyr(t-Bu)-OH tR = 11.40(16%). FMOC-Tyr(t-Bu)-Cl(methyl ester) tR = 14.42 (80%).

## EXAMPLE 2

### Synthesis of Leucine Enkephalin

A) From Acid Chloride via manual synthesis.--Reactions were performed in syringes as described above.

1 g of FMOC-Leu-resin (0.1 meq/g)
0.2 M DIEA/HOBt solution
4 equiv. of FMOC-AA-Cl
FMOC-Tyr(t-Bu)-Cl 191 mg
FMOC-Gly-Cl 126.2 mg
FMOC-Gly-Cl 126.2 mg
FMOC-Phe-Cl 162.4 mg
Theoretical yield = 66.9 mg.

After synthesis, the resin was treated with TFA (20 mL, 2h), filtered, the filtrate evaporated to dryness and 50 mL of dry ether added to precipitate the peptide. After filtration, washing, drying and lyophilization, there was obtained 53 mg (79.2%) of the leucine enkephalin. GC 10.87 (Gly), 12.66 (D-Leu, 1.42%), 13.69 (Leu), 22.66 (D-Phe, 1.1%), 23.17 (Phe), 28.14 (Tyr). In the GC trace an unidentified peak appeared at 15 min. This appeared to be derived from solvent or derivatization reagents, as it was not observed in all runs. HPLC f=1, (MeOH/$H_2O$ .1% TFA, 50:50), tR=10.26 (75.4% pure). Amino acid analysis (theoretical) Gly 1.9 (2), Phe 0.8 (1), Tyr 0.8 (1), Leu 0.9(1).

D-$Phe^4$-Leucine Enkephalin was prepared following the same procedure from 400 mg of FMOC-Leu-resin. After TFA (10 mL, 2h), treatment and precipitation, there was obtained 17 mg (61.2%) of the pentapeptide. GC 10.986 (Gly), 12.71 (D-Leu, 1.46%), 13.76 (Leu), 22.80 (D-Phe), 23.32 (Phe, 0.67%), 27.74 (D-Tyr, 1.1%), 28.15 (Tyr). In the GC trace an unidientified peak appeared at 15 min. This appeared to be derived from the solvent or derivatization reagents as it was not observed in all runs. HPLC f=1

($MeOH/H_2O$ .1% TFA, 50:50), tR = 17.90 (79.76% pure). Amino acid analysis (theoretical) Gly 1.7 (2), Phe 0.9 (1), Tyr 0.9(1), Leu 0.9(1).

B) From Acid Chloride via the Pepsynthesizer (Milligen 9050).

Resin subsitution = 01. meq/g
Resin quantity = 1.0 g
Fmoc-Phe-Cl 0.203 g
Fmoc-Gly-Cl 0.158 g
Fmoc-Gly-Cl 0.158 g
Fmoc-Tyr(t-Bu)-Cl 300 mg
DIEA/HOBt/DMF solution (0.33 M)

| General Instrument Protocol: | | |
|---|---|---|
| Step Type | Flow Rate (mL/min) | Duration |
| 01 DMF wash | 3.0 | 00:00:30 |
| 02 PiP wash | 9.2 | 00:04:00 |
| 03 DMF wash | 13.4 | 00:05:00 |
| 04 Dissolve AA | 0.0 | 0 |
| 05 Wait for liquid handler | 1.0 | 0 |
| 06 DMF wash | 3.0 | 00:00:10 |
| 07 Flush probe | 0.0 | 0 |
| 08 Wait for liquid handler | 0.0 | 0 |
| 09 DMF + HOBt/DIEA wash probe | 10.0 | 00:00:15 |
| 10 Recycle | 3.0 | 00:00:10 |
| 11 Recycle with loop | 5.0 | 0 |
| 12 Recycle | 13.4 | 00:10:00 |
| 13 DMF wash loop | 13.4 | 00:03:00 |
| 14 Flush probe | 0.0 | 0 |
| 15 Wait for liquid handler | 0.0 | 0 |
| 16 DMF + HOBt/DIEA wash probe | 10.0 | 00:01:00 |
| 17 DMF wash | 3.0 | 00:00:02 |
| 18 Flush probe | 0.0 | 20 |
| 19 Wait for liquid handler | 0.0 | 0 |
| 20 Rinse probe | 0.0 | 0 |
| 21 Wait for liquid handler | 1.0 | 0 |

After synthesis, the resin was treated with TFA (20 mL, 2h), to give 46 mg (68.7%) of the crude peptide, 93% pure (HPLC), GC 10.67 (Gly), 12.53 (D-Leu, 1.4%), 13.55 (Leu), 23.10 (Phe), 28.13 (Tyr).

DL-$Phe^4$ Leucine Enkephalin was synthesized similarly except that near-racemic phenylalanine was used and tyrosine was added as pfp ester rather than acid chloride.

Resin subsitution = 0.1 meq/g
Resin quantity = 1.0 g
Fmoc-DL-Phe-Cl 0.203 g
Fmoc-Gly-Cl 0.158 g
Fmoc-Gly-Cl 0.158 g
Fmoc-Tyr(t-Bu)-OPFP 0.313 g

After TFA treatment (20 mL, 2 h) there was obtained 50 mg (74.7%) of the crude peptide 98.7% pure, (HPLC). The L-Phe and D-Phe diastereomers were present in the ratio 45/53 (HPLC).

C) From PFP Esters via the Pepsynthesizer (Milligen 9050).

D-$Phe^4$-Leucine Enkephalin was synthesized according to the standard 30-min acylation protocol from 1 g of FMOC-Leu-resin. After TFA (20 mL, 2 h) treatment, there was obtained 41 mg (59%) of the crude peptide. GC 10.922 (Gly), 12.69 (D-Leu, 1.62%), 13.72 (Leu), 22.74 (D-Phe), 23.24 (Phe, 1.06%), 27.77 (D-Tyr, 0.85%), 28.16 (Tyr). HPLC f = 1, ($MeOH/H_2O$) .1% TFA, 50:50) tR = 17.80 (95.3% pure). Amino acid analysis (theoretical) Gly 2(2), Phe 0.9(1), Tyr 0.9 (1), Leu 0.8(1).

D-$Tyr^5$-Leucine Enkephalin was prepared according to the standard 30-min. acylation protocol from 1 g

of Fmoc-Leu-resin. After TFA (20 mL, 2h) treatment, there was obtained 36 mg (53.8%) of the pentapeptide. HPLC f = 1, (MeOH/$H_2$OH/$H_2$O .1% TFA. 50:50)tR = 9.70 (87.2% pure).

D) From PFP Esters by Using DIEA/HOBt

Fmoc-Phe-OPFP 0.277 g-Bu)-OPFP 0.313 g

Fmoc-Gly-OPFP 0.232 g

Fmoc-Gly-OPFP 0.232 g

Fmoc-Tyr(t-Bu)-OPFP 0.313 g

After TFA treatment, there was obtained 50 mg (74.7%) of the crude peptide 100% pure (HPLC).

Stability of FMOC-AA-Resin in the Presence of HOBt/t-Amine Mixtures.--Tests were carried out on the stability toward deblocking of FMOC amino acid-linked resins by the HOBt/amine mixtures. Although unhindered bases caused deblocking`the hindered base, di-isopropyl ethyl amine (DIEA) was without effect for up to 4 hours which is far longer than the times required for acylation. Thus deblocking during acylation is not expected to be a problem. Results are recorded in Table II.

Table 2

| *Question of Possible Premature Deblocking via Catalyst/Base Mixtures* | | | |
|---|---|---|---|
| Fmoc-AA-resin | HOBt/amine | Time | Kaiser Test |
| Leu | HOBt/TEA | 30 min | + |
| Leu | HOBt/DIEA | 30 min | - |
| ILe | HOBt/DIEA | 4 h | - |
| ILe | HOBt/NMM | 4 h | - |
| ILe | HOBt/DMAP | 4 h | + |

As shown in Example 1, Fmoc-Phe-Cl was coupled with a leucine-loaded resin as follows.

Scheme 2

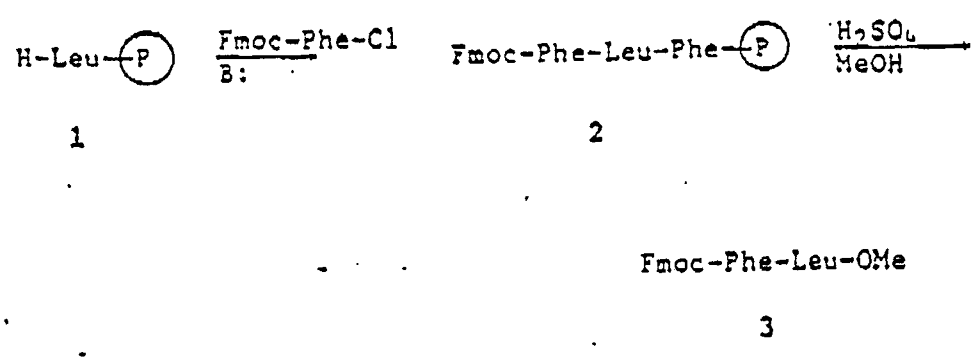


1

2

3

wherein P = polymeric resin support.

The resulting dipeptide resin 2 was subjected to transesterification in methanol in the presence of a small amount of sulfuric acid to give the protected dipeptide methyl ester 3 (85-90%). HPLC examination of the crude material showed less than 0.1% of the DL diastereomer.

This method is also applicable to the synthesis of an actual peptide using the Milligen 9050 continuous flow peptide synthesizer. As as example, leucine enkephalin 4 was synthesized by the process of the present invention in Example 2.

H-Tyr-Phe-Gly-Gly-Leu-OH  4

For the first synthesis an acylation time of 10 min. was arbitrarily set to allow a significant safety

margin, although this may be longer than necessary. This leads to a complete cycle time of 25 min, in contrast to the one-hour cycle time recommended for the normal protocol. Five molar equivalents of Fmoc-AA-Cl were used along with an equimolar amount of the mixture of the present invention. The four coupling steps (Fmoc-Gly-Cl twice, Fmoc-Phe-Cl, Fmoc-Tyr-($CMe_3$)-Cl) were effected automatically, the pentapeptide being obtained in a yield of 69%. HPLC analysis showed the crude product to have a purity of 93%. Thus the crude product is of higher purity than that obtained by the normal technique with pfp esters using one-hour cycle times. Even better was a "mixed" synthesis with acid chlorides used throughout with the exception of the Tyr pfp ester: yield 75%, purity 99%. No trace (0.1%) of the D-$Phe^4$ or D-$Tyr^5$-diastereomers was detected showing clearly that racemization is not a problem using these acid chlorides under appropriate reaction conditions.

Another striking observation was then made: reaction of pfp esters was also catalysed by the mixture used in the present process and cycle times were as short as when acid chlorides were used. A similar synthesis of leucine enkephalin was carried out using commercially available pfp esters in place of acid chlorides. In this case a 10-min acylation time was also used and the only difference was the slightly longer time needed to dissolve and draw up the pfp esters which are significantly less soluble than acid chlorides. Remarkably, the pentapeptide was obtained in a yield of 75% and a purity of 100%, even higher than observed with the acid chlorides.

Thus the mixture of the present process is advantageous not only in allowing the synthesis of high purity peptides via inexpensive acid chlorides, but also makes possible the synthesis of super-pure peptides via the previously- recommended but more expensive pfp esters. For the synthesis of extra pure crude peptides one may be willing to accept the higher cost of pfp esters over acid chlorides. Because both substrates can be used with only one minor change in protocol, it is also possible to program the instrument easily for continuous, automated "mixed" synetheses. For example, in cases where the Fmoc amino acids carry t-butyl-based side chain protecting groups, acid chlorides may be contraindicated because of their expected instability on long-term storage. In fact some such compounds, including Fmoc-Tyr-($CMe^3$)-Cl, are slowly degraded on standing with loss of the t-butyl moiety. In other cases the t-butyl derivatives are oils (Ser,Thr) rather than crystalline solids. Such amino acids are therefore best introduced via pfp esters even while the majority of the simple amino acids are introduced via acid chlorides. Other acid chlorides in this category are those from Glu, Gln, Asp, Asn and Arg. In this way any peptide should be obtainable with the appropriate combination of pfp ester and acid chloride. Typically about 75% of the amino acids will be available in acid chloride form. It may also be noted that eventually substitution of t-butyl side chain protection by an appropriate alternate protective system should make it possible to use acid chlorides for every amino acid. The pfp ester technique would then be reserved for cases where either (a) high purity peptides are desired, or (b) longer peptides are being synthesized where the higher coupling yields at each step will ensure cleaner reactions for such longer sequences.

Other embodiments are given in the following examples.

## EXAMPLE 3

General protocol for manual synthesis of H-Val-Gln-Ala-Ile-Asp-Tyr-Ile-Asn-Gly-OH

The reactions described hereinbelow were carried out manually in small syringes with mixing being accomplished by brief stirring by hand. The syringe was attached to a Millipore vacuum manifold to effect rapid removal of reagents and solvents. The Fmoc-Gly-PepKa resin was first washed with DMF, deblocked with piperidine, and then acylated according to the 6 step-protocol reproduced below. In each cycle for 1 g of FMOC-Gly PepKa (0.1 mmol), there was used 4 equivalents of Fmoc-AA-acylating agent (used in the order given below) and 5 ml of 0.08M HOBt/DIEA solution.

6- step protocol

1) DMF was 10 mL (1 min x 1)
2) 20% piperidine/DMF wash (5 min x 2)
3) DMF wash 10 mL (2 min x 5)
4) HOBt/DIEA wash 5 mL (2 min x 1)

5) Acylation

6) DMF wash 10 mL (2 min x 3)

The AA acylating agents were added as follows:

Fmoc-Asn (ONP) (190 mg, 30 min), Fmoc-Ile-Cl (149 mg, 15 min), Fmoc-Tyr (t-Bu)-opfp(250 mg, 15 min), Fmoc-Asp (O-t-Bu)-opfp, (230 mg, 15 min), Fmoc-Ile-Cl (149 mg, 15 min); Fmoc-Ala-Cl (132 mg, 15 min), Fmoc-Gln-ONP (196 mg, 1 h), Fmoc-Val-Cl (143.2 mg, 45 min, double acylation) still gave positive Kaiser test).

Theoretical yield (as mono TFA salt):120 mg

Amount obtained:94.4 mg (78.6)

The product formed was relatively pure.

## EXAMPLE 4

The procedure of Example 3 was repeated except the acid chloride were replaced by PFP esters. The amount of product obtained was 94.4 mg (78.6%).

The product formed was relatively pure.

## EXAMPLE 5

Synthesis of H-Ala-Asn-Lys-Gly-Phe-Leu-Glu-Glu-Val-OH

The procedure was as in Example 4

(A) 1.5 g of Fmoc-Val-PepSyn-KA (0.135 mmol)

4 eq of appropriate FMOC-AA-OPfp ester

6 mL of 0.1 M HOBt/DIEA solution except for the case Fmoc-Asn-OPfp where HOBt alone was used

Fmoc-Glu(O t Bu)-OPfp (319 mg, 15 min), FMOC-Glu(O t -Bu)-OPfp (319 mg, 15 min), Fmoc-Leu-OPfp (281 mg, 15 min), FMmoc-Phe-OPfp (299 mg, 15 min), Fmoc-Gly-OPfp (250 mg, 15 min), Fmoc-Lys-(BOC)-OPfp (343 mg, 15 min), Fmoc-Asn-OPfp (281 mg, 30 min), Fmoc-Ala-OPfp (258 mg, 15 min)

Theoretical yield (as di TFA salt): 166.59 mg

Amount obtained: 120 mg (72%)

The product formed was relatively pure.

(B) 1.0 g of Fmoc-Val-PepSyn-KA (0.09 mmol)

4 eq of acylating agent (acid chloride where possible otherwise pfp or ONp esters)

5 mL of 0.08 M HOBt/DIEA solution

Fmoc-Glu(O t Bu)-OPfp (213 mg, 10 min), Fmoc-Glu(O t Bu)-OPfp (213 mg, 10 min), Fmoc-Leu-Cl (134 mg, 16 min), Fmoc-Phe-Cl (146 mg, 10 min), Fmoc-Gly-Cl (114 mg, 10 min), Fmoc-Lys(BOC)-OPfp (228 mg, 10 min), Fmoc-Asn-ONp (179 mg, 30 min recoupled), Fmoc-Ala-Cl (119 mg, 10 min) Theoretical yield (as di TFA salt): 111.06 mg

Amount obtained: 74 mg (66.75%)

The product formed was relatively pure.

## EXAMPLE 6

Decomposition of Asn-Pfp Ester:

0.5 g (0.956 mmol) of FMOC-Asn-OPfp in 5 mL of DMF was checked by TLC from time to time. After stirring at room temperature for 36 h only a trace of starting material was left. The resulting solution was poured into 50 mL of water, the precipitate extracted with EtOAC, and the organic solution washed with saturated $NaHCO_3$ and NaCl solution. The organic solution was dried over $MgSO_4$ and evaporated to give a residue which was recrystallized from EtOAc-Skelly B to give 70 mg (22%) of Fmoc-Asu; IR (KBr) 3337,

3200 (NH), 1787, 1717 $cm^{-1}$ (C=O). The aqueous solution was acidified with conc. HCl to pH 3 and extracted with EtOAc. After drying and removal of solvent, there was obtained 154 mg (48%) of Fmoc- β -(CN)-Ala-OH; IR (KBr 3316 (NH), 2258 (CN), 1717 $cm^{-1}$ (C=O) along with a little FMOC-Asn-OH.

When Fmoc-Asn-OPfp was allowed to stand in HOBt/DIEA/DMF solution, after 15 min there was formed a significant amount of Fmoc-Asu and FMOC- β -(CN)-Ala-OH according to TLC analysis.

## EXAMPLE 7

### Speed of Coupling Reactions in the Presence of Various t-Amine and N-Hydroxy Compounds

200 mg of appropraite FMOC-AA-PepSyn-KA (0.1 mmol/g loading)
4 eq of acylating agent
0.5 mL of 0.16 M binary salt or t-amine alone
The acylating agent was dissolved in 0.3 mL of DMF solution and the final conc. was 0.1 M
Quantitative analysis was determined by UV absorption of

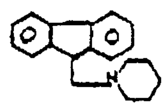

(A) FMOC-Val-Cl ---Leu-PepSyn-KA---> FMOC-Val-Leu-PepSyn-KA

| Base | Coupling Time (min) | Coupling (%) |
|---|---|---|
| Pyridine | 10 | 61.9 |
| 2,6-di-t-butyl pyridine | 10 | 35.3 |
| DIEA | 10 | 89.5 |
| HOSu/DIEA | 3 | 14.8 |
| Phthalimide-N-OH/DIEA | 3 | 19.47 |
| HOOBT/DIEA | 1 | 74.1 |
| | 2 | 93.7 |
| | 3 | Negative Kaiser Test |
| HOBt/DIEA | 1' | 97.34 |
| | 1 | Negative Kaiser Test |
| | | Negative Kaiser Test |

(B) FMOC-Val-OPfp ---Leu-PepSyn-KA---> FMOC-Val-Leu-PepSyn-KA

| | | |
|---|---|---|
| HOCBt.DIEA | 1 | 29 |
| | 2 | 95 |
| | 3 | Negative Kaiser Test |
| HOBt/DIEA | 1 | 94 |
| | 2 | Negative Kaiser Test |
| | 3 | Negative Kaiser Test |

FMOC-Ile-OPfp ---Ile-PepSyn-KA---> FMOC-ILe-ILe-PepSyn-KA

| | | |
|---|---|---|
| HOBt/DIEA | 1 | 94.4 |
| | 2 | 97.85 |
| | 3 | Negative Kaiser Test |
| | 6 | Negative Kaiser Test |
| HOBt | 3 | 36.9 |
| | 7 | 58 |
| | 10 | 71.54 |
| | 15 | 79 |
| HOOBt | 3 | 55.4 |
| | 7 | 83.25 |
| | 10 | 92.5 |
| | 15 | 96.2 |

(D) FMOC-Leu-ONp ---Ile-PepSyn-KA---> FMOC-Leu-Ile-PepSyn-KA

| | | |
|---|---|---|
| HOBt/DIEA | 10 | Negative Kaiser Test |

| | | |
|---|---|---|
| FMOC-Asn-ONp | | |
| HOBt/DIEA | 10 | Negative Kaiser Test |

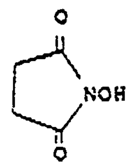 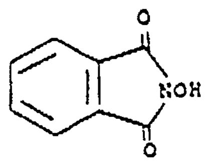 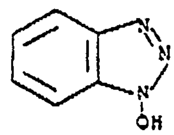 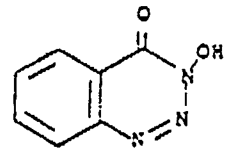

HOSu phthalimide-N-OH HOBt HOOBt

EXAMPLE 8

Synthesis of Tetraethylammonium Benzotriazole 1-Oxide:

To a solution of 10 mL of 1.5 M of tetraethyl ammonium hydroxide was added 2.3 g of HOBt.$H_2O$. The resulting solution was stirred at room temperature for 1 hour, solvent evaporated, the residue dried with the aid of vacuo pump for 4 hours and then recrystallized from $CH_2Cl_2$/hexane to give 1 g (26%) of the product as a white solid, mp 107-115 °C; NMR ($CDCl_3$) δ 1.2 (t, $CH_3$), 3.2 (q, $CH_2$), 7.2-7.8 (m, aryl).

EXAMPLE 9

Synthesis of DMAP.2HOBt Salt:

To a solution of 244 mg (2 mmole) of DMAP in 20 mL of $CH_2Cl_2$ was added 306 mg (2 mmole) of HOBt.$H_2O$. The resulting solution was stirred for 15 min, the solvent evaporated, and the residue recrystallized from acetone/ether to give 294 mg (75%) of the product as white crystals, mp 97-98 °C; NMR ($CDCl_3$) δ 3.1 (s, $CH_3$), 6,6 (d, aryl), 7.2-7.8 (m, aryl), 8.2 (d, aryl).

| | | | |
|---|---|---|---|
| Anal. Calcd for $C_{19}H_{20}N_8O_2$: | C, 58.15; | H, 5.13; | N, 28.55. |
| Found: | C, 57.88; | H, 4.98; | N, 28.04. |

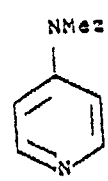

DMAP

EXAMPLE 10

Synthesis of DMAP.2HOOBt Salt:

To a solution of 244 mg (2 mmole) of DMAP in 20 mL of MeOH was added 326 mg (2mmole) of HOOBt. The resulting solution was stirred for 30 min, solvent evaporated, the yellow residue recrystallized from Ether/MeOH to give 233 mg (52%) of the product as yellow crystals, mp 199-200 °C; NMR (DMSO) δ 3.1 (s, $CH_3$), 6.9 (d, aryl), 7.7-8.2 (m, aryl).

| | | | |
|---|---|---|---|
| Anal. Calcd for $C_{21}H_{20}N_8O_4$: | C, 56.24; | H, 4.49; | N, 24.98. |
| Found: | C, 56.05; | H, 4.54; | N, 24.79. |

EXAMPLE 11

Synthesis of DABCO.2HOOBt Salt:

To a solution of 244 mg (2mmole) of DABCO in 20 mL of dry $CH_2Cl_2$ was added 326 mg (2 mmole) of HOOBt. The resulting solution was stirred for 30 min, solvent evaporated, the yellow residue recrystallized from acetone/ether to give 100 mg (22.8%) of the product as a yellow solid, mp 172-174 °C; NMR ($CDCl_3$) δ 3.25 (s, $CH_2$), 7.6-8.4 (m, aryl).

| | | | |
|---|---|---|---|
| Anal. Calcd for $C_{20}N_{22}N_8O_4$: | C, 54.79; | H, 5.05; | N, 25.55. |
| Found: | C, 54.58; | H, 5.04; | N, 25.27. |

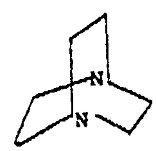

DABCO

The above preferred embodiments and examples are given to illustrate the scope and spirit of the present invention. These embodiments and examples will make apparent, to those skilled in the art, other embodiments and examples. These other embodiments and examples are within the contemplation of the present invention. Therefore, the present invention should be limited only by the appended claims.

## Claims

1. A process for forming a peptide bond between a first AMINO ACID containing a free N-terminal amino group and an acylating N-terminal amino protected second AMINO ACID which comprises reacting under amide forming conditions said first AMINO ACID with said second AMINO ACID in the presence of a basic and catalytic effective amount of a mixture comprising a tertiary amine of the formula:

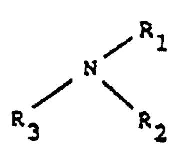

and a N-hydroxy nitrogen containing heterocycle or the quaternary ammonium salt of said mixture, wherein

$R_1$, $R_2$, and $R_3$ are independently lower alkyl, or $R_1$ and $R_2$ together with the nitrogen to which they are attached form a heterocyclic ring.

2. A process for preparing a peptide which comprises

(a) reacting a first AMINO ACID containing a free N-terminal amino group and an acylating N -amino protected amino acid under amide forming conditions in the presence of basic and catalytically effective amount of a tertiary amine of the formula:

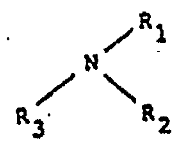

and a N-hydroxy nitrogen containing heterocycle or the quaternary ammonium salt of said mixture, wherein

$R_1$, $R_2$, $R_3$ are independently lower alkyl, or $R_1$ and $R_2$ together with the nitrogen to which they are attached form a heterocyclic ring;

(b) removing the Nα-protecting groups from the amino acid residue;

(c) repeating steps (a) and (b) until the desired peptide has been obtained.

3. The process according to Claims 1 or 2 wherein the second AMINO ACID is an acylating Nα-amino-protected α-amino acid.

4. The process according to any of Claims 1 to 3 wherein the protecting group is a base labile Nα-amino acid protecting group, a nucleophile labile Nα-amino acid protecting group, an acid labile Nα-amino acid protecting group, or a Nα-amino acid protecting group which is removable by hydrogenation.

5. The process according to any of Claims 1 to 4 wherein the amino acid has the formula:

L - AA - X

wherein L is a base lablile Nα-amino acid protecting group, a nucleophile labile Nα-amino acid protecting group, an acid labile Nα-amino acid protecting group or a Nα-amino acid protecting group removable by hydrogenation;

AA is an amino acid residue; and

X is a chloro, bromo or

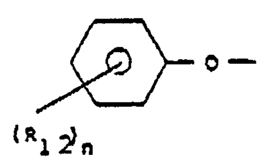

wherein each $R_{12}$ is independently halo, nitro or cyano,

and n is an integer from 0-5.

6. The process according to any of Claims 1-5 wherein L is 9-fluoroenylmethyloxycarbonyl, 2-(t-butylsulfonyl)-2-propenyloxycarbonyl, benzothiophene sulfone-2-methyloxycarbonyl, t-butyloxycarbony, or benzyloxy carbonyl.

7. The process according to any of Claims 1-6 wherein the second AMINO ACID is Fmoc-AA-chloride or Fmoc-AA-pentafluorophenyl ester, wherein AA is one of the twenty natural occurringα-amino acids.

8. The process according to any of Claims 1-7 wherein the N-hydroxide of a nitrogen containing heterocyclic compound is

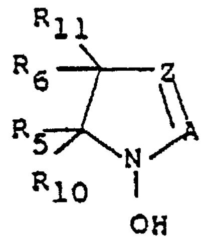 , 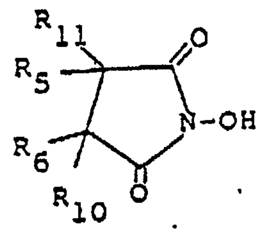 ,

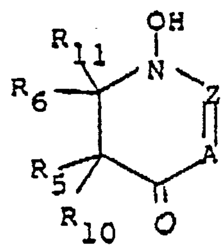 , 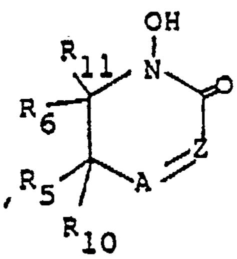 , or

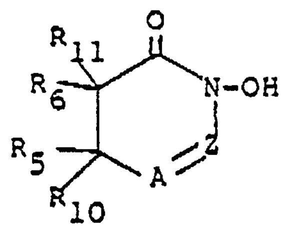

wherein

$R_6$ and $R_5$ are independently hydrogen or lower alkyl or $R_6$ and $R_5$ taken together with the carbon atoms to which they are attached form an aromatic ring which is unsubstituted or monosubstituted with lower alkyl or an electron withdrawing group;

$R_{10}$ and $R_{11}$ are hydrogen or taken together form a bond, or $R_{10}$ and $R_{11}$ are , when $R_5$ and $R_6$ taken together form an aromatic ring;

A is N or $CR_7$;

Z is N or $CR_8$ and

A is N when Z is N; and

$R_7$ and $R_8$ are independently hydrogen, lower alkyl, or an electron withdrawing group.

9. The process according to any of Claims 1-8 wherein the N-hydroxide of the N-containing heterocycle is

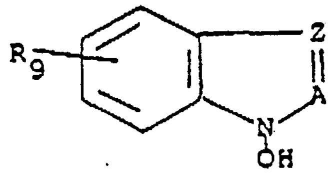 , 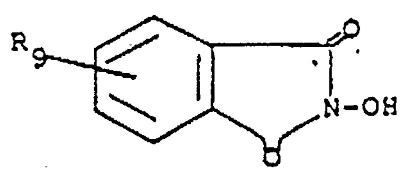 ,

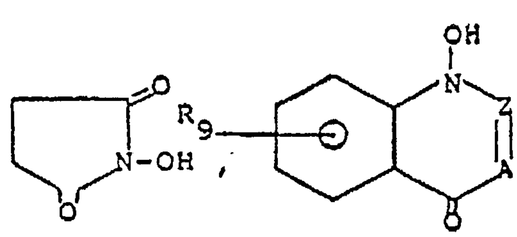 , 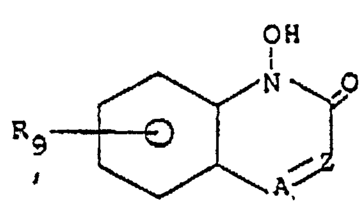 , or

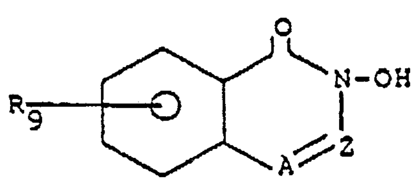 .

wherein
A is N or $CR_7$;
Z is N or $CR_8$; and
$R_7$ and $R_8$ and $R_9$ are independently hydrogen, lower alkyl or an electron withdrawing group.

10. The process according to any of Claims 8-9 wherein the electron withdrawing group is nitro or trifluoromethyl.

11. The process according to any of Claim 1-10 wherein the N-hydroxide of the nitrogen containing heterocycle is

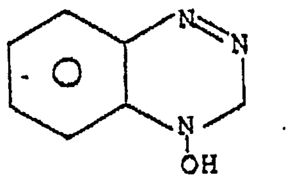
,
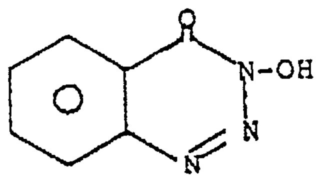
or
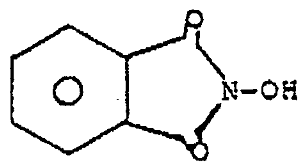
,
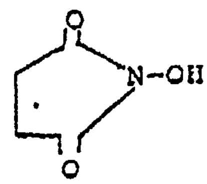
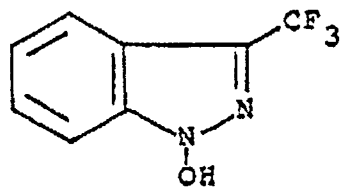
,

12. The process according to any of Claims 1-11 wherein the tertiary amine is diisopropyl ethylamine, triethylamine N-methylmorpholine, DMAP or DABCO.

13. The process according to any of Claims 1-12 wherein the salt of the mixture has the formula:

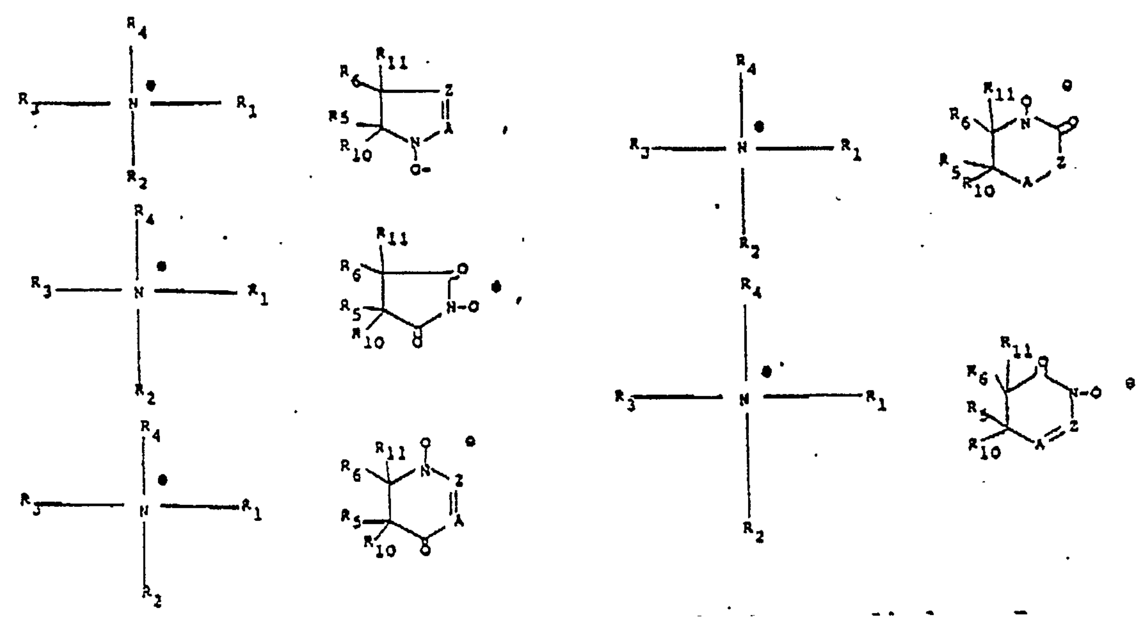

wherein $R_1$, $R_2$, $R_3$ and $R_4$ are independently lower alkyl or $R_1$ and $R_2$ taken together with the nitrogen to which they are attached form a heterocycle;
$R_6$ and $R_5$ are independently hydrogen or lower alkyl or $R_6$ and $R_5$ taken together with the carbon atoms to which they are attached form an aromatic ring which is unsubstituted or monosubstituted with lower alkyl or

an electron withdrawing group;
$R_{10}$ and $R_{11}$ are hydrogen or taken together form a bond, or $R_{10}$ and $R_{11}$ are , when $R_5$ and $R_6$ taken together form an aromatic ring;
A is N or $CR_7$;
Z is N or $CR_8$; and
A is N when A is N;
$R_7$ and $R_8$ is hydrogen or lower alkyl or an electron withdrawing group; and
$R_4$ is hydrogen or alkyl containing $C_1$-$C_{20}$ carbon atoms.

14. The process according to any of Claims 1-13 wherein $R_5$ and $R_6$ taken together with the carbon to which they are attached form a phenyl ring.

15. The process according to any of Claims 1-14 wherein the salt is $Et_4N^+$ $^-OBt$, an ammonium salt of DMAP and HOBt; an ammonium salt of DMAP and HOOBt, an ammonium salt of DABCO and HOOBt, or an ammonium salt of HOBt and diisopropylamine.

16. The process according to any of Claims 1-15 wherein the N-hydroxy nitrogen containing heterocycle and tertiary amine is present in approximately equimolar amounts.

17. The process according to any of Claims 1-16 wherein the second AMINO ACID, the N-hydroxy-N-containing heterocycle and the tertiary amine are present in equimolar amounts.

18. The process according to any of Claims 1-17 wherein a dehydrating agent is additionally present.

19. The process according to Claim 18 wherein the dehydrating agent is dicyclohexycarbodiimide.

20. The process according to any of Claims 1-19 wherein the mixture is an equimolar amount of hydroxybenzotriazole and diisopropylethyl amine.

21. The process according to any of Claims 1-20 wherein the process is carried out in dimethylformamide.

22. The process according to any of Claims 1-21 wherein the first AMINO ACID is covalently coupled to a solid phase peptide synthesis resin and the desired peptide is cleaved from the resin.

23. The process according to any of Claims 1-22 wherein the tertiary amine is diisopropyl ethyl amine and the N-hydroxy-N-containing heterocycle is hydroxybenzotriazole, N-hydroxy(oxo)benzotriazine or

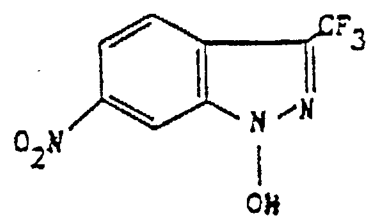

24. The process according to any of Claims 1-23 wherein the mixture is $(Et)_4{}^+N\ OBt^-$.

25. A quaternary ammonium salt comprising as a base, an oxide of a N-hydroxy containing heterocycle and as the acid, a quaternary ammonium cation of the formula:

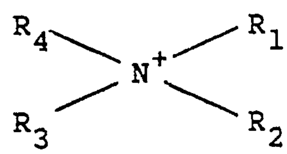

wherein $R_1$ and $R_2$ and $R_3$ are independently lower alkyl or $R_1$ and $R_2$ taken together with the nitrogne to which they are attched form a heterocyclic ring; and
$R_4$ is hydrogen, or alkyl containing from 1 to 20 carbon atoms.

26. The salt according to claim 25 wherein the salt has the formula:

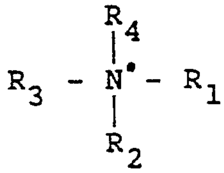 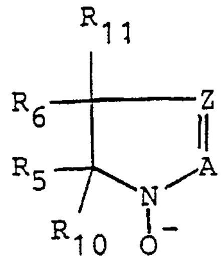

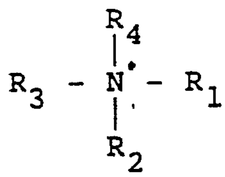 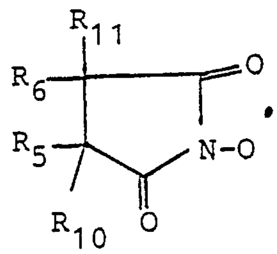

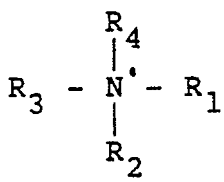 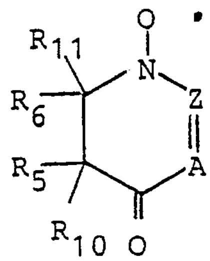

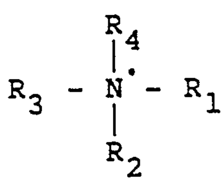 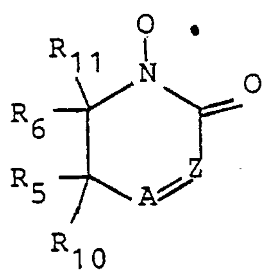

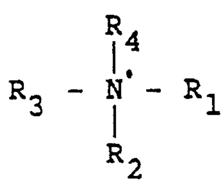 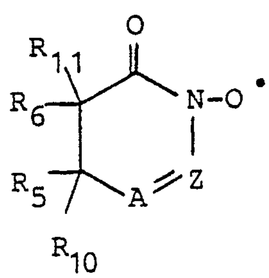

wherein

$R_1$, $R_2$ and $R_3$ are independently lower alkyl or $R_1$ and $R_2$ taken together with the nitrogen to which they are attached form a heterocycle;

$R_6$ and $R_5$ are independently hydrogen or lower alkyl or $R_6$ and $R_5$ taken together with the carbon atoms to which they are attached form an aromatic ring which is unsubstituted or monosubstituted with lower alkyl or an electron withdrawing group;

A is N or $CR_7$;

Z is N or $CR_8$ and

A is N when Z is N;

$R_7$ and $R_8$ are independently hydrogen, lower alkyl or an electron withdrawing group and
$R_4$ is hydrogen or alkyl containing from 1 to 20 carbon atoms;
$R_{10}$ and $R_{11}$ are hydrogen or taken together form a bond, or $R_{10}$ and $R_{11}$ are , when $R_6$ and $R_5$ taken together form an aromatic ring.

27. The salt according to Claims 25-26 wherein $R_{10}$ and $R_{11}$ taken together form a bond.

28. The salt according to Claims 25-27 wherein $R_5$ and $R_6$ taken together with the carbon to which they are attached form a phenyl ring.

29. The salt according to Claims 25-28 wherein the cation diisopropylethylammonium, $(DMAP)^{+2}$, $(DABCO)^{+2}$, $Et_4N^+$, or $Et_3NH^+$.

30. The salt according to Claims 25-29 which is tetraethylammonium benzotriazole 1-oxide, DMAP.2HOBt, DMAP.2HOOBt, DABCO.2HOOBt, DIEA.HOBt, or $Et_3N$-HOBt.

SEQUENCE LISTING

SEQ ID No: 1

SEQUENCE TYPE: AMINO ACID SEQUENCE

SEQUENCE LENGTH: 5 AMINO ACID RESIDUES

TOPOLOGY: LINEAR

MOLECULE TYPE: PEPTIDE

IMMEDIATE EXPERIMENTAL SOURCE: SYNTHETIC

NAMED: LEUCINE ENKEPHALIN 4

```
Tyr-Phe-Gly-Gly-Leu          5
 1              5
```

SEQ ID No: 2

SEQUENCE TYPE: AMINO ACID SEQUENCE

SEQUENCE LENGTH: 9 AMINO ACID RESIDUES

TOPOLOGY: LINEAR

MOLECULE TYPE: PEPTIDE

SOURCE: SYNTHETIC

```
Val-Gln-Ala-Ile-Asp-Tyr-Ile-Asn-Gly    9
 1               5
```

SEQ ID No: 3

SEQUENCE TYPE: AMINO ACID SEQUENCE

SEQUENCE LENGTH: 9 AMINO ACID RESIDUES

TOPOLOGY: LINEAR

MOLECULE TYPE: PEPTIDE

SOURCE: SYNTHETIC

```
(3):      Ala-Asn-Lys-Gly-Phe-Leu-Glu-Glu-Val      9
           1                5
```